Europäisches Patentamt

European Patent Office

Office européen des brevets

Publication number: **0 319 890**
**A1**

# EUROPEAN PATENT APPLICATION

(21) Application number: 88120281.6

(22) Date of filing: 05.12.88

(51) Int. Cl.⁴: **C07H 15/10 , A61K 37/22 , A61K 35/30**

(30) Priority: 09.12.87 IT 2291187

(43) Date of publication of application:
14.06.89 Bulletin 89/24

(84) Designated Contracting States:
AT BE CH DE ES FR GB GR IT LI LU NL SE

(71) Applicant: Crinos Industria Farmacobiologica S.p.A.
Piazza XX Settembre, n. 2
I-22079 Villa Guardia Como(IT)

(72) Inventor: Fedeli, Gianfranco
Via Zante 19
I-20100 Milano(IT)
Inventor: Diamantini, Giuseppe
Via Ranuzzi 10
I-61032 Fano (Pesaro)(IT)

(74) Representative: Dragotti, Gianfranco et al
SAIC BREVETTI s.r.l. Viale Bianca Maria, 15
I-20122 Milano(IT)

(54) A process for the preparation of monosialoganglioside.

⑤ By carrying out the autolysis of beef brain under particular conditions all gangliosides contained in the animal tissues are reduced to monosialoganglioside without degradation of the latter.

EP 0 319 890 A1

## A process for the preparation of monosialoganglioside

The present invention relates to the production of monosialoganglioside. This substance is normally prepared by extraction from the nervous tissues of mammalians, especially from the brain of the total gangliosides; from the latter the monosialoganglioside can be then separated by chromatography on the DEAE Sephadex or on silica gel.

The extraction of the total gangliosides is carried out by processes basically based on the repartition technique already described by Folch et al. (J. Biol. Chem. 226,497,1967) and repeated with several variations by a number of authors and in a number of Patents.

The chromatographic separation of the single gangliosides described in a number of literature papers, is possible but it not suitable for the requirements of an industrial preparation owing to the complexity of the elution systems, to the great volume of the solvents required for small product masses and, mainly, owing to the difficulties which are met in order to made a very complex chromatographic system industrially reproductable. Moreover it is to be considered that the content of monosialoganglioside is only 15-20% of the total gangliosides, whereby the yield of these processes are a relevant economical problem. Otherwise, as an alternative, it is also possible to reduce to mono- sialoganglioside all the gangliosides of a tissue by incubating them with sialidases (bacterial or tissue neuraminidases) by which can be attacked by enzymatic way all the sialic residues of these molecules apart from that of the monosialoganglioside GM1 which, in the absence of surface active substances, cannot be attacked by such an enzyme (Sugano et al. FEBS Lett. 98, 321,1978). This enzymatic treatment would have the advantage of increasing the yields, since all the gangliosides would be converted into monosialoganglioside thus relevantly semplifying the chromtographic purification steps.

As a matter of fact also this process is practiceable only for small laboratory preparations. The reason is that of the high cost of the purified enzyme and of its lability, which requires cautions and cares which cannot be easily adopted in an industrial production. Moreover the tissutal neuraminidases are often not pure owing to the presence of galactosidases which may compromise the basic oligosaccharide structure of the ganglioside.

The main purpose of the present invention is that of providing an industrially applicable and advantageous process for the production of monosialoganglioside which is devoid of the problems and of the drawbacks of the previously known processes.

A more specific purpose of the present invention is that of providing a process for the industrial production with high yields of monosialoganglioside starting from animal organs.

It has been now found and it is the object of the present invention that by operating under particular conditions of autolysis it is possible, starting from beef brain of the type normally collected in the slaughter hauses and stored in the refrigerators, to reduce all the gangliosides present in the tissue to monosialoganglioside without the degradation of the latter, in spite of the presence of surface active agents addded to the treated mass.

The process according to the present invention is characterized more specifically by the following step sequence:

a) grinding the whole organ and homogeneizing it in water containing bacteriostatic agents:

b) then adding an homogeneously dispersed surface active agent;

c) suspending the homogeneizate in buffer at pH of between 4 and 5 and adjusting the mass to a temperature of between 15 and 25°C;

d) Maintaining the mass under stirring under the above conditions for the time needed to reduce all the gangliosides to monosialoganglioside (from 2 to 16 hours);

e) Collecting and washing with water the solid material separated by filtration;

f) Dehydrating and defatting the solid mass with acetone.

The resulting material, thus dehydrated and defatted, is subjected to ex traction and purification of the gangliosides with known technics, such as for instance the extraction and the repartition according to Folch, the dialysis and the chromatography on the DEAE Sephadex, as for instance described by Masao Iwamori et al. (Biochim. Biophis. Acta 528, 257,1978). The process of the invention leads to yields of monosialoganglioside of between 1 and 2 grams per kg. of brain, whereas in the previous processes 0.2-0.5 grams per Kg it might be obtained.

In the process according to the present invention the surface active agent added in the step (b) is selected among the non ionic ones, named octoxinoles, water miscible, such as for example Triton X-100 or Igepal CA-650.

In turn the preliminarily used bacteriostatic agent is selected among acetates, the phenolic compounds. whereas the buffer used in the step (c) is selected among acetates and ftalates.

An example of preparation carried out in pilot plant is hereinafter decribed for illustrating and non limiting purpose.

200 Kg of frozen beef brain, of food quality, have been ground in meat grinding machine and suspended in 380 litres of softened water containing 0.5% of phenol.

To the mass, maintained under stirring, 960 litres of softened water containing 4.8 litres of Triton X-100 (product sold by Rohm and Haas as surface active agent) have been added.

After 15 minutes of stirring 100 litres of 4.5M acetate buffer have been added at PH 4.35.

The temperature has been adjusted to 18° C.

Through a preliminary test, carried out on a small amount of the same batch of organs, an autolysis time of 6 hours has been determined; consequently the mass has been maintained under the above conditions for 6 hours.

After that time 29 Kg of filtration adjuvant have been added (Clarcel FLO/MA) and the mixture has been filtered on a filterpress whereby the liquid has been removed.

The solid residue has been then dispersed in 400 litres of water and filtered again on filterpress, the liquid being always removed.

The solid material has been dispersed in 300 litres of anhydrous acetone for 15 minutes.

The suspension has been filtered on industrial Buchner. The solid residue has been dispersed and filtered two times again with 300 litres of anhydrous acetone each time.

The solid material has been finally dried under vacuum leading to about 45 Kg of powder containing 1.5 gr. of N-acetil-neuraminic acid per Kg corresponding to 1.7 gr. of monosialoganglioside per Kg. of organ being processed.

The mass of solid material has been extracted with 300 litres of a cloroform: metanol (2:1) mixture for two times. The liquids have been filtered and combined, the solid being removed.

The filtered liquids have been treated with 120 litres of an aqueous solution of 0.1M sodium chloride and then the repartition as described by Folch has been carried out.

After this treatment 642 g. of raw monosialoganglioside have been obtained. having a title of 10.1% of N-acetil-neuraminic acid corresponding to a yield of 1.62 g. of monosialoganglioside per Kg of organ processed.

The raw ganglioside has been subsequentely purified by ionic exchange chromatography on DEAE Sephadex A25 according to the technique described by Masao and Nagaj.

The final solution, purified by dialysis and filtered through a membrane with 0.22 microns of porosity has been lyophilized.

There have been thus obtained 276 g of monosialoganglioside having the following characteristics:

| bonded N-acetyl-neuraminic acid | 20,6% |
|---|---|
| freed N-acetil-neuraminic acid | 0.01% |
| glycerophosphatidic phosphor | 0.03% |

chromatographic composition:

monosialoganglioside GM1 = 94%
monosialoganglioside GM2 = 6%

## Claims

1) A process for the industrial production of monosialoganglioside starting from beef brain, characterized by the steps of:
   a) grinding the whole organ and homogeneizing it in water containing bacteriostatic agents:
   b) then adding an homogeneously dispersed surface active agent;

3

c) suspending the homogeneizate in buffer at pH of between 4 and 5 and adjusting the mass to a temperature of between 15 and 25°C;

d) Maintaining the mass under stirring under the above conditions for the time needed to reduce all the gangliosides to monosialoganglioside (from 2 to 16 hours);

e) Collecting and washing with water the solid material separated by filtration;

f) Dehydrating and defatting the solid mass with acetone.

2) A process according to claim 1, characterized in that said surface active agent is selected among the non ionic detergents miscible with water.

3) A process according to claim 1, characterized in that said step (c) has a duration of between 2 and 16 hours.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | FR-A-2 585 707 (MITSUI TOATSU CHEMICALS INC.) * Whole document; in particular page 5, lines 12-34 * | 1 | C 07 H 15/10 A 61 K 37/22 A 61 K 35/30 |
| Y | BIOCHEMISTRY, vol. 17, no. 13, 27th June 1978, pages 2619-2628, American Chemical Society; L.O. SILLERUD et al.: "Assignment of the 13C nuclear magnetic resonance spectrum of aqueous ganglioside GM1 micelles" * Whole document * | 1 | |
| A | BIOCHIMICA ET BIOPHYSICA ACTA, vol. 296, 1973, pages 160-170, Elsevier. Sc. Publ. Co., Amsterdam, NL; G. TETTAMANDI et al.: " A new procedure for the extraction, purification and fractionation of brain gangliosides" * Pages 162-164 * | 1-3 | |
| A,D | FEBS LETTERS, vol. 89, no. 2, May 1978, pages 321-325; K. SUGANO et al: "Susceptibility of ganglioside GM1 to a new bacterial neuraminidase" * Pages 324-325 * | 1-3 | **TECHNICAL FIELDS SEARCHED (Int. Cl.4)** A 61 K 31/00 C 07 H 15/00 A 61 K 35/00 A 61 K 37/00 |
| A | FR-A-2 319 399 (INSTITUT MERIEUX) * Page 14, example 3 * | 1-3 | |
| A | EP-A-0 016 702 (INSTITUT NERIEUX) * Page 2, lines 13-22 * | 1-3 | |

The present search report has been drawn up for all claims

| Place of search | Date of completion of the search | Examiner |
|---|---|---|
| THE HAGUE | 27-02-1989 | GERLI P.F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P0401)